# EUROPEAN PATENT APPLICATION

(11) **EP 4 066 868 A1**
(43) Date of publication of application: **05.10.2022**
(21) Application number: 20909754.2
(22) Date of filing: 26.10.2020
(51) Int. Cl.: A61L 27/36, A61L 27/50, A61L 27/58

(54) **COLLAGEN SCAFFOLD PREPARATION METHOD AND COLLAGEN SCAFFOLD**

(30) Priority: 30.12.2019 CN 201911394705
(71) Applicant: Wan, Mianshui, Shantou, Guangdong 515041 (CN)
(72) Inventor: LIN, Chuangxin, Shantou, Guangdong 515041 (CN); WAN, Mianshui, Shantou, Guangdong 515041 (CN)
(74) Representative: Wang, Bo
(86) International application number: PCT/CN2020/123741
(87) International publication number: WO 2021/135563

(57) **Abstract**

The present invention provides a method for preparing a collagen scaffold and the collagen scaffold. The method for preparing a collagen scaffold comprises: cleaning a raw material tissue; performing freeze-thaw cycle treatment on the cleaned raw material tissue to obtain a first intermediate; degreasing the first intermediate to obtain a second intermediate; decellularizing the second intermediate to obtain a third intermediate; removing nucleic acids from the third intermediate to obtain a fourth intermediate; and washing and drying the fourth intermediate to obtain the collagen scaffold. The nucleic acids are removed using an omnipotent nuclease solution with a content of 100 U/L to 10,000 U/L. The collagen scaffold prepared by the method provided in the present application can promote the proliferation and migration of seed cells, improve the stability of expressions of the seed cells according to an original phenotype, and promote the secretion of a cell matrix to increase the firmness of the scaffold.

## Description

### TECHNICAL FIELD

The present invention relates to the technical field of tissue engineering, and in particular to a method for preparing a collagen scaffold and the collagen scaffold.

### BACKGROUND

Tissue engineering is an emerging interdisciplinary science that involves cell biology and material science and constructs tissues or organs in vitro or in vivo. A basic principle of the tissue engineering is as follows: a small amount of living tissue is obtained from the body, cells (also called seed cells) are separated from the tissue using a special enzyme or other methods, the separated cells are cultured and expanded in vitro, the expanded cells are mixed with a biodegradable and absorbable biomaterial (scaffold) with a good biocompatibility in a certain proportion, such that the cells are adhered to the biomaterial (scaffold) to form a cell-material complex; and the complex is implanted into a lesion part of a tissue or an organ of the body, the biomaterial is gradually degraded and absorbed in the body, the implanted cells continuously proliferate in the body, secrete extracellular matrix and form the corresponding tissue or organ. Therefore, the purposes of repairing wound and reconstructing functions are achieved. A three-dimensional structure formed by the biomaterial scaffold provides a good environment for cells to acquire nutrients, grow and metabolize.

The biomaterial scaffold, namely a collagen scaffold, prepared by an existing method has a small total surface area and poor structural reliability. In addition, the existing method can only process and prepare single raw material tissues, and thus has a small application range.

### SUMMARY

Based on the above situation, the present invention mainly aims to provide a method for preparing a collagen scaffold to solve the above problems in the existing preparation method.

To achieve the foregoing objectives, the present invention uses the following technical solutions:
A first aspect of the present invention provides a method for preparing a collagen scaffold, including the following steps:
S100, cleaning a raw material tissue;
S200, performing freeze-thaw cycle treatment on the cleaned raw material tissue to obtain a first intermediate;
S300, degreasing the first intermediate to obtain a second intermediate;
S400, decellularizing the second intermediate to obtain a third intermediate;
S500, removing nucleic acids from the third intermediate to obtain a fourth intermediate; and
S600, washing and drying the fourth intermediate to obtain the collagen scaffold; and
   where in the step S500, the nucleic acids are removed using an omnipotent nuclease solution with a content of 100 U/L to 10,000 U/L, such as 100 U/L, 500 U/L, 1,000 U /L, 1,500 U/L, 2,000 U/L, 2,500 U/L, 3,000 U/L, 3,500 U/L, 4,000 U/L, 4,500 U/L, 5,000 U/L, 5,500 U/L, 6,000 U /L, 6,500 U/L, 7,000 U/L, 7,500 U/L, 8,000 U/L, 8,500 U/L, 9,000 U/L, 9,500 U/L and 10,000 U/L.

The raw material tissue is subjected to freeze-thaw cycle treatment, ice is formed in a freeze-thaw cycle treatment process and a concentration of a salt solution is changed through the freeze-thaw cycle treatment (a solubility of salt in unfrozen water will change as a temperature drops during the freeze-thaw cycle treatment, and the concentration of the salt gradually increases, generating a concentration difference between an inside and an outside of a cell membrane), thus cells are ruptured, the cell rupture effect is improved, a larger total surface area is obtained, and regeneration of tissues is facilitated.

Since a main difference of different raw material tissues lies in a difference of DNA and RNA. A specific step of removing nucleic acids is contained in the preparation method of the present application. In the step, the Omnipotent nuclease is used to remove the nucleic acids, thus degrading DNA and RNA in all forms, has strong universality and a wide application range, and is suitable for combined treatment of different single raw material tissues and different raw material tissues. The raw material tissues may be a tissue or an organ of mammals such as pigs, cattle, and sheep suitable as a raw material, such as heart, liver, kidneys, bladder, diaphragm, omentum majus, mesentery.

Preferably, in the step S200, the cleaned raw material tissue is added to a buffer containing tris(hydroxymethyl)aminomethane and phenylmethylsulfonyl fluoride at pH 7.8 to 8.2, such as 7.8, 7.9, 8, 8.1 and 8.2, to perform the freeze-thaw cycle treatment.

The buffer can improve the cell rupture effect and the phenylmethylsulfonyl fluoride can provide a stable environment for the tissue to prevent a protease released in cells from affecting other protein structures (constituting the structure of a matrix material) of the raw material tissue, thus a three-dimensional space of the generated collagen scaffold is more stable.

Preferably, in the buffer, the tris(hydroxymethyl)aminomethane has a molar concentration of 1 mmol/L to 20 mmol/L, such as 1 mmol/L, 2 mmol/L, 3 mmol/L, 4 mmol/L, 5 mmol/L, 6 mmol/L, 7 mmol/L, 8 mmol/L, 9 mmol/L, 10 mmol/L, 11 mmol/L, 12 mmol/L, 13 mmol/L, 14 mmol/L, 15 mmol/L, 16 mmol/L, 17 mmol/L, 18 mmol/L, 19 mmol/L and 20 mmol/L, more preferably 8 mmol/L to 12 mmol/L, still more preferably 10 mmol/ L; and the phenylmethylsulfonyl fluoride has a mass percentage of 0.1% to 1%, such as 0.1%, 0.2%, 0.3%, 0.4%, 0.5%, 0.6%, 0.7%, 0.8%, 0.9% and 1%, more preferably 0.8% to 1%, still more preferably 1%.

Preferably, the buffer may also contain ethylenediaminetetraacetic acid and the ethylenediaminetetraacetic acid has a molar concentration of 1 mmol/L to 10 mmol/L, such as 1 mmol/L, 2 mmol/L, 3 mmol/L, 4 mmol/L /L, 5 mmol/L, 6 mmol/L, 7 mmol/L, 8 mmol/L, 9 mmol/L and 10 mmol/L, more preferably 8 mmol/L to 10 mmol/L, still more preferably 10 mmol/L.

The ethylenediaminetetraacetic acid is used to adjust a pH value of the buffer to be within a range of 7.8 to 8.2. In addition, since the ethylenediaminetetraacetic acid has an inhibitory effect, when being used to adjust the pH value, it can avoid damaging other protein structures (constituting the structure of the collagen scaffold) of the raw material tissue and thus further ensures that the three-dimensional space of the generated collagen scaffold is more stable.

Preferably, in the freeze-thaw cycle treatment, a first freeze-thaw cycle treatment includes freezing at a temperature of -80°C to -20°C, such as -80°C, -75°C, -70°C, -65°C, -60°C, -55°C, -50°C, -45°C, -40°C, -35°C, -30°C, -25°C and -20°C, for 1h to 8 h, such as 1 h, 2 h, 3 h, 4 h, 5 h, 6 h, 7 h and 8 h; and thawing at a temperature of 30°C to 40°C, such as 30°C, 31°C, 32°C, 33°C, 34°C, 35°C, 36°C, 37°C, 38°C, 39°C and 40°C, for 0.5 h to 1 h, such as 0.5 h, 0.6 h, 0.7 h, 0.8 h, 0.9 h and 1 h.

Preferably, the freeze-thaw cycle treatment may be performed 2-3 times, more preferably 3 times.

Although the freeze-thaw cycle treatment is an existing processing technology, for the collagen scaffold having a relatively strict demand, an excessive high thaw temperature will lead to protein denaturation and an excessive low thaw temperature will lead to incomplete thawing. Besides, since the half-life of the phenylmethylsulfonyl fluoride as a protease inhibitor is too short, if thawing time is too long, an intracellular protease after the cell rupture will enzymatically hydrolyze an extracellular matrix. Therefore, the technology is not used in a preparation process of the existing collagen scaffold. In the present application, the temperatures and time are limited, thus the freeze-thaw cycle treatment can well rupture the cells without damaging the extracellular matrix.

Preferably, the step S300 includes the following steps:
S310, washing the first intermediate using ethanol in a low-to-high concentration gradient;
S320, degreasing the tissue washed in the step S310 using acetone and hexane; and
S330, washing the tissue treated in the step S320 using ethanol in a high-to-low concentration gradient to obtain the second intermediate.

In the step S310, the tissue can be dehydrated, the washing with ethanol in the low-to-high concentration gradient can avoid that a direct use of high-concentration ethanol makes the cells rapidly lose water to form a dense layer and affect the dehydration effect. In the step S330, the degreased tissue can be hydrated to restore an original state of the matrix and protect original properties of the matrix.

Preferably, the step S310 includes the following steps:
S311, washing the first intermediate using ethanol at a concentration of 50% to 70%, such as 50%, 55%, 60%, 65% and 70%, to obtain an intermediate I; and
S312, washing the intermediate I using ethanol at a concentration of 99% to 100%, such as 99%, 99.2%, 99.4%, 99.6%, 99.8% and 100%, to obtain an intermediate II.

Preferably, in the step S311, the first intermediate is washed once for 0.5 h to 1 h, such as, 0.5 h, 0.6 h, 0.7 h, 0.8 h, 0.9 h and 1 h.

Preferably, in the step S312, the intermediate I is washed 2 to 3 times for 0.5 h to 1 h, such as, 0.5 h, 0.6 h, 0.7 h, 0.8 h, 0.9 h and 1 h, each time.

In the step S311, dehydration is achieved. In the step S312, water in the tissue can be taken away as much as possible by washing with high-concentration ethanol, thus incomplete degreasing caused by formation of water-in-oil (that is, a solvent surrounds a water-containing tissue) during a subsequent degreasing process is prevented.

Preferably, the step S320 includes the following steps:
S321, extracting polar fats from the intermediate II using acetone to obtain an intermediate III; and
S322, extracting non-polar fats from the intermediate III using a mixture of hexane and acetone to obtain an intermediate IV.

Preferably, in the step S321, the intermediate II is washed using the acetone at a concentration of 99% to 100%, 1 to 3 times for 0.5 h to 1 h, such as, 0.5 h, 0.6 h, 0.7 h, 0.8 h, 0.9 h and 1 h, each time.

Preferably, in the step S322, the intermediate III may be washed 1 to 3 times with a mixture of hexane and acetone for 4 h to 16 h, such as 4 h, 5 h, 6 h, 7 h, 8 h, 9 h, 10 h, 11 h, 12 h, 13 h, 14 h, 15 h and 16 h, each time.

Preferably, in the step S322, the hexane and the acetone have a mass ratio of (1-4):(1-3), such as 1:1, 1:2, 1:3, 2:1, 2:3, 3:1, 3:2, 4:1 and 4:3. A combination of the hexane and the acetone at the ratio range can rapidly and efficiently extract the non-polar fats.

Preferably, the step S330 includes the following steps:
S331, washing the intermediate IV using ethanol at a concentration of 99% to 100%, such as 99%, 99.2%, 99.4%, 99.6%, 99.8% and 100%, to obtain an intermediate V; and
S332, washing the intermediate V using ethanol at a concentration of 50% to 70%, such as 50%, 55%, 60%, 65% and 70%, to obtain the second intermediate.

In the step S331, the hexane and the acetone can be washed away using the high-concentration ethanol so as to facilitate a hydration process of the tissue using the low-concentration ethanol in the step S332.

Preferably, in the step S331, the intermediate IV is washed once for 0.5 h to 1 h.

Preferably, in the step S332, the intermediate V is washed once at a temperature of 2°C to 40°C for 8 h to 24 h, such as 8 h, 10 h, 12 h, 14 h, 16 h, 18 h, 20 h, 22 h and 24 h. Since the ethanol has a penetrating power stronger than water, the dehydration process is faster than the hydration process and the duration of the step S332 is longer.

Preferably, the step S400 includes the following steps:
S410, washing the second intermediate using a phosphate-buffered saline at pH 7 to 8.2, such as 7, 7.2, 7.4, 7.6, 7.8, 8 and 8.2, to obtain an intermediate A; where the ethanol remaining in the previous step can be removed through the step;
S420, washing the intermediate A using a solution containing tris(hydroxymethyl)aminomethane and ethylenediaminetetraacetic acid to obtain an intermediate B;
S430, washing the intermediate B using a solution containing sodium lauryl sulfate to obtain an intermediate C;
S440, washing the intermediate C using a phosphate-buffered saline containing sodium deoxycholate to obtain an intermediate D; and
S450, washing the intermediate D using a phosphate-buffered saline to obtain the third intermediate.

Preferably, in the step S410, the second intermediate is washed 1 to 4 times for 0.5 h to 1 h, such as 0.5 h, 0.6 h, 0.7 h, 0.8 h, 0.9 h and 1 h, each time.

Preferably, in the step S420, the tris(hydroxymethyl)aminomethane has a content of 1 mmol/L to 20 mmol/L, such as 1 mmol/L, 3 mmol/L, 5 mmol/L, 7 mmol/L, 9 mmol/L, 11 mmol/L, 13 mmol/L, 15 mmol/L, 17 mmol/L, 19 mmol/L and 20 mmol/L; the ethylenediaminetetraacetic acid has a content of 1 mmol/L to 10 mmol/L, such as 1 mmol/L, 2 mmol/L, 3 mmol/L, 4 mmol/L, 5 mmol/L, 6 mmol/L, 7 mmol/L, 8 mmol/L, 9 mmol/L and 10 mmol /L; and the washing is performed for 0.5 h to 4 h, such as 0.5 h, 1 h, 1.5 h, 2 h, 2.5 h, 3 h, 3.5 h and 4 h. A low concentration of Tris-EDTA enables tissue cells to be supplemented with water by an osmotic pressure and relaxation of the tissues is more conducive to the subsequent decellularization.

Preferably, in the step S430, the sodium lauryl sulfate has a mass percentage of 0.5% to 1.5%, such as 0.5%, 0.6%, 0.7%, 0.8%, 0.9%, 1%, 1.1%, 1.2%, 1.3%, 1.4% and 1.5%, and washing is performed 2 to 3 times for 8 h to 24 h, such as 8 h, 10 h, 12 h, 14 h, 16 h, 18 h, 20 h, 22 h and 24 h, each time.

Preferably, in the step S440, the sodium deoxycholate has a content of 1 mmol/L to 10 mmol/L, such as 1 mmol/L, 2 mmol/L, 3 mmol/L, 4 mmol/L, 5 mmol/L, 6 mmol/L, 7 mmol/L, 8 mmol/L, 9 mmol/L and 10 mmol/L; and the washing is performed 1 to 3 times for 0.5 h to 2 h, such as 0.5 h, 0.6 h, 0.7 h, 0.8 h, 0.9 h, 1 h, 1.1 h, 1.2 h, 1.3 h, 1.4 h, 1.5 h, 1.6 h, 1.7 h, 1.8 h, 1.9 h and 2 h, each time.

The sodium lauryl sulfate and the sodium deoxycholate can lyse cells and nuclear membranes, and the combination of the two can effectively remove cytoplasmic proteins in dense tissues.

Preferably, in the step S450, the washing is performed 2 to 3 times for 0.5 h to 2 h, such as 0.5 h, 0.6 h, 0.7 h, 0.8 h, 0.9 h, 1 h, 1.1 h, 1.2 h, 1.3 h, 1.4 h, 1.5 h, 1.6 h, 1.7 h, 1.8 h, 1.9 h and 2 h, each time.

The phosphate-buffered saline is used to wash away the residual sodium lauryl sulfate and sodium deoxycholate.

Preferably, the step S500 includes the following steps:
S510, washing the third intermediate using a buffer containing tris(hydroxymethyl)aminomethane and a magnesium salt at pH 7.8 to 8.2, such as 7.8, 7.9, 8, 8.1 and 8.2 to obtain an intermediate a; and
S520, at a temperature of 30°C to 42°C, such as 30°C, 31°C, 32°C, 33°C, 34°C, 35°C, 36°C, 37°C, 38°C, 39°C, 40°C, 41°C and 42°C, washing the intermediate a using a solution containing tris(hydroxymethyl)aminomethane, magnesium salt, bovine serum albumin and Omnipotent nuclease at pH 7.8 to 8.2, such as 7.8, 7.9, 8, 8.1 and 8.2 to obtain the fourth intermediate.

Preferably, in S510, the tris(hydroxymethyl)aminomethane has a content of 30 mmol/L to 60 mmol/L, such as 30 mmol/L, 35 mmol/L, 40 mmol/L, 45 mmol/L, 50 mmol/L mmol/L, 55 mmol/L and 60 mmol/L; the magnesium salt has a content of divalent magnesium ion of 1 mmol/L to 10 mmol/L, such as 1 mmol/L, 2 mmol/L, 3 mmol/L, 4 mmol/L, 5 mmol/L, 6 mmol/L, 7 mmol/L, 8 mmol/L, 9 mmol/L and 10 mmol/L; and the washing is performed for 0.5 h to 2 h.

Preferably, in S520, the tris(hydroxymethyl)aminomethane has a content of 30 mmol/L to 60 mmol/L, such as 30 mmol/L, 35 mmol/L, 40 mmol/L, 45 mmol/L, 50 mmol/L, 55 mmol/L and 60 mmol/L; the magnesium salt has a content of divalent magnesium ion of 1 mmol/L to 10 mmol/L, such as 1 mmol/L, 2 mmol/L, 3 mmol/L, 4 mmol/L, 5 mmol/L, 6 mmol/L, 7 mmol/L, 8 mmol/L, 9 mmol/L and 10 mmol/L; the bovine serum albumin has a mass percentage of 0.01% to 1%, such as 0.01%, 0.05%, 0.1%, 0.15%, 0.2%, 0.25%, 0.3%, 0.35%, 0.4%, 0.45%, 0.5%, 0.55%, 0.6%, 0.65%, 0.7%, 0.75%, 0.8%, 0.85%, 0.9 %, 0.95% and 1%; and the washing is performed for 8 h to 48 h, such as 8 h, 10 h, 15 h, 20 h, 25 h, 30 h, 35 h, 40 h, 45 hand 48 h.

A cell material is concentrated in the nucleus and thus has a very high concentration therein. Therefore, a hypertonic Tris solution is used. The Omnipotent nuclease can degrade all forms of DNA and RNA. The bovine serum albumin (BSA) can improve the enzyme activity and enable digestion to be more complete. Meanwhile, the activity of the nuclease depends on the presence of Mg²⁺.

Preferably, the step S600 includes the following steps:
S610, washing the fourth intermediate using a solution containing tris(hydroxymethyl)aminomethane at pH 7.8 to 8.2, such as 7.8, 7.9, 8, 8.1 and 8.2 to obtain an intermediate b, where the tris(hydroxymethyl)aminomethane can wash away the nuclease, the bovine serum albumin, an enzymatic hydrolysis product and the magnesium ion;
S620, washing the intermediate b using a phosphate-buffered saline at pH 7 to 8.2, such as 7, 7.2, 7.4, 7.6, 7.8, 8 and 8.2, to obtain an intermediate c, where the phosphate-buffered saline can wash away the tris(hydroxymethyl)aminomethane and substances that cannot be washed away by the tris(hydroxymethyl)aminomethane in the step S610;
S630, washing the intermediate c using double distilled water to obtain an intermediate d, where the double distilled water can wash away the phosphate-buffered saline; and
S640, freeze-drying the intermediate d to obtain the collagen scaffold.

Preferably, the washing in the step S610 is performed 1 to 3 times for 0.5 to 2 h, such as 0.5 h, 0.6 h, 0.7 h, 0.8 h, 0.9 h, 1 h, 1.1 h, 1.2 h, 1.3 h, 1.4 h, 1.5 h, 1.6 h, 1.7 h, 1.8 h, 1.9 h and 2 h, each time.

Preferably, the washing in the step S620 is performed 1 to 3 times for 0.5 to 2 h, such as 0.5 h, 0.6 h, 0.7 h, 0.8 h, 0.9 h, 1 h, 1.1 h, 1.2 h, 1.3 h, 1.4 h, 1.5 h, 1.6 h, 1.7 h, 1.8 h, 1.9 h and 2 h, each time.

Preferably, the washing in the step S630 is performed 1 to 3 times for 0.5 to 2 h, such as 0.5 h, 0.6 h, 0.7 h, 0.8 h, 0.9 h, 1 h, 1.1 h, 1.2 h, 1.3 h, 1.4 h, 1.5 h, 1.6 h, 1.7 h, 1.8 h, 1.9 h and 2 h, each time.

Preferably, in the washing process, unless otherwise specified, the phosphate-buffered saline (PBS) has a pH of 7 to 8.2, such as 7, 7.2, 7.4, 7.6, 7.8, 8 and 8.2, and the washing process is performed at a temperature of 0-40°C in a shaking box or an orbital shaker at 50-200 rpm.

Experiments show that the collagen scaffold prepared by the preparation method provided in the present application can promote the proliferation and migration of seed cells, improve the stability of expressions of the seed cells according to an original phenotype, and promote the secretion of a cell matrix to increase the firmness of the scaffold. In addition, the collagen scaffold also has good biocompatibility, good degradation performance and strong integration ability to hosts.

A second aspect of the present invention provides a collagen scaffold prepared by the preparation method.

### BRIEF DESCRIPTION OF THE DRAWINGS

Exemplary embodiments of the present invention are described below with reference to the accompanying drawings.

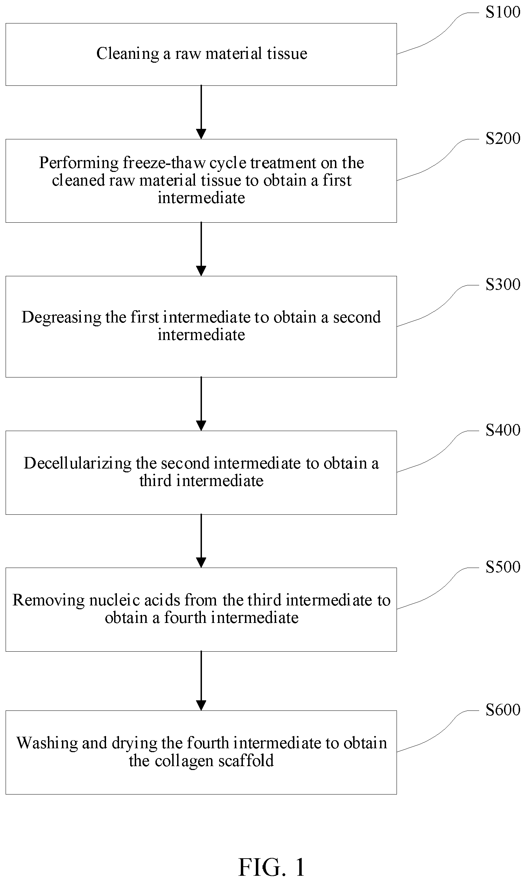

FIG. 1 shows a flow chart of a method for preparing the collagen scaffold provided by the present invention.

### DETAILED DESCRIPTION

The present invention is described below based on examples, but the present invention is not limited to these examples only. In the following detailed description of the present invention, some specific details are described in detail. In order to avoid obscuring the essence of the present invention, well-known methods, procedures, procedures and elements are not described in detail.

In addition, a person of ordinary skill in the art should understand that the accompanying drawings provided herein are for illustrative purposes and are not necessarily drawn to scale.

Unless the context clearly requires otherwise, throughout the specification and claims, words such as "including", "comprising", and the like should be construed in an inclusive rather than an exclusive or exhaustive sense; that is, in the sense of "including but not limited to".

In the description of the present invention, it should be understood that the terms "first", "second" and the like are used for descriptive purposes only, and should not be construed as indicating or implying relative importance. In addition, in the description of the present invention, unless otherwise specified, "plurality" means two or more.

Aiming at problems that an existing method for preparing a collagen scaffold has a small application range and the prepared collagen scaffold has a relatively small total surface area and a poor structural reliability, the present invention provides a method for preparing a collagen scaffold and the collagen scaffold prepared by the preparation method. As shown in FIG. 1, the preparation method includes the following steps:
S100, cleaning a raw material tissue;
S200, performing freeze-thaw cycle treatment on the cleaned raw material tissue to obtain a first intermediate;
S300, degreasing the first intermediate to obtain a second intermediate;
S400, decellularizing the second intermediate to obtain a third intermediate;
S500, removing nucleic acids from the third intermediate to obtain a fourth intermediate; and
S600, washing and drying the fourth intermediate to obtain the collagen scaffold; and
   in the step S500, the nucleic acids are removed using an omnipotent nuclease and the Omnipotent nuclease has a content of 100 U/L to 10,000 U/L.

Specific examples of the preparation method are given below:

### Embodiment 1

Fresh pig omentum majus was washed 3 times with normal saline.

The washed omentum majus was added to a buffer containing 10 mmol/L tris(hydroxymethyl)aminomethane (Tris), 10 mmol/L ethylenediaminetetraacetic acid (EDTA), and phenylmethylsulfonyl fluoride (PMSF) at a mass percentage of 1% at pH 8.0 for 3 freeze-thaw cycle treatments. The first freeze-thaw cycle treatment was performed at a freezing temperature of -80°C for 1 h and a thawing temperature of 37°C for 0.5 h.

The tissue treated by the freeze-thaw cycle treatment was degreased:
washing was performed using ethanol at a concentration of 50% for 0.5 h;
washing was performed using ethanol at a concentration of 99% twice for 0.5 h each time;
washing was performed using acetone at a concentration of 99% once for 0.5 h;
washing was performed using a solution of hexane and acetone at a mass ratio of 1:1 once for 4 h;
washing was performed using ethanol at a concentration of 99% for 0.5 h; and
washing was performed using ethanol at a concentration of 50% at a temperature of 2°C for 8 h.

The degreased tissue was decellularized:
washing was performed using a phosphate-buffered saline at pH 7 once for 0.5 h;
washing was performed using a solution containing 1 mmol/L tris(hydroxymethyl)aminomethane and 1 mmol/L ethylenediaminetetraacetic acid for 0.5 h;
washing was performed using a solution containing sodium lauryl sulfate at a mass percentage of 0.5% once for 8 h;
washing was performed using a phosphate-buffered saline containing 1 mmol/L sodium deoxycholate once for 0.5 h; and
washing was performed using a phosphate-buffered saline once for 0.5 h.

Nucleic acids were removed from the degreased tissue:
washing was performed using a buffer containing 30 mmol/L tris(hydroxymethyl)aminomethane and 1 mmol/L magnesium ion-containing magnesium salt at pH 7.8 for 0.5 h; and
washing was performed using a solution containing 30 mmol/L tris(hydroxymethyl)aminomethane, 1 mmol/L magnesium ion-containing magnesium salt, bovine serum albumin at a mass percentage of 0.01% and 100 U/L Omnipotent nuclease at pH 7.8 at a temperature of 30°C for 8 h.

After the nucleic acids were removed, the tissue was washed and freeze-dried:
washing was performed using a solution, containing 30 mmol/L tris(hydroxymethyl)aminomethane at pH 7.8 once for 0.5 h;
washing was performed using a phosphate-buffered saline at pH 7 once for 0.5 h;
washing was performed using double distilled water once for 0.5 h; and freeze-drying was performed to obtain a collagen scaffold.

### Embodiment 2

A fresh pig heart tissue was washed 3 times with normal saline.

The washed heart tissue was added to a buffer containing 1 mmol/L tris(hydroxymethyl)aminomethane (Tris), 1 mmol/L ethylenediaminetetraacetic acid (EDTA), and phenylmethylsulfonyl fluoride (PMSF) at a mass percentage of 0.1% at pH 7.8 for 3 freeze-thaw cycle treatments. The first freeze-thaw cycle treatment was performed at a freezing temperature of -20°C for 8 h and a thawing temperature of 30°C for 1 h.

The tissue treated by the freeze-thaw cycle treatment was degreased:
washing was performed using ethanol at a concentration of 70% for 1 h;
washing was performed using ethanol at a concentration of 100% 3 times for 1 h each time;
washing was performed using acetone at a concentration of 100% 3 times for 1 h;
washing was performed using a solution of hexane and acetone at a mass ratio of 4:3 3 times for 16 h;
washing was performed using ethanol at a concentration of 100% for 1 h; and
washing was performed using ethanol at a concentration of 70% at a temperature of 40°C for 24 h.

The degreased tissue was decellularized:
washing was performed using a phosphate-buffered saline at pH 8.2 4 times for 1 h each time;
washing was performed using a solution containing 20 mmol/L tris(hydroxymethyl)aminomethane and 10 mmol/L ethylenediaminetetraacetic acid for 4 h;
washing was performed using a solution containing sodium lauryl sulfate at a mass percentage of 1.5% 3 times for 24 h each time;
washing was performed using a phosphate-buffered saline containing 10 mmol/L sodium deoxycholate 3 times for 2 h each time; and
washing was performed using a phosphate-buffered saline 3 time for 2 h each time.

Nucleic acids were removed from the degreased tissue:
washing was performed using a buffer containing 60 mmol/L tris(hydroxymethyl)aminomethane and 10 mmol/L magnesium ion-containing magnesium salt at pH 8.2 for 2 h; and
washing was performed using a solution containing 60 mmol/L tris(hydroxymethyl)aminomethane, 10 mmol/L magnesium ion-containing magnesium salt, bovine serum albumin at a mass percentage of 1% and 10,000 U/L Omnipotent nuclease at pH 8.2 at a temperature of 42°C for 48 h.

After the nucleic acids were removed, the tissue was washed and freeze-dried:
washing was performed using a solution containing 60 mmol/L tris(hydroxymethyl)aminomethane at pH 8.2 3 times for 2 h each time;
washing was performed using a phosphate-buffered saline at pH 8.2 3 times for 2 h each time;
washing was performed using double distilled water 3 times for 2 h each time; and freeze-drying was performed to obtain a collagen scaffold.

### Embodiment 3

A fresh pig bladder tissue was washed 3 times with normal saline.

The washed bladder tissue was added to a buffer containing 20 mmol/L tris(hydroxymethyl)aminomethane (Tris), 8 mmol/L ethylenediaminetetraacetic acid (EDTA), and phenylmethylsulfonyl fluoride (PMSF) at a mass percentage of 0.8% at pH 8.2 for 3 freeze-thaw cycle treatments. The first freeze-thaw cycle treatment was performed at a freezing temperature of -50°C for 5 h and a thawing temperature of 40°C for 0.8 h.

The tissue treated by the freeze-thaw cycle treatment was degreased:
washing was performed using ethanol at a concentration of 60% for 0.8 h;
washing was performed using ethanol at a concentration of 99.5% twice for 0.8 h each time;
washing was performed using acetone at a concentration of 99.5% twice for 0.8 h;
washing was performed using a solution of hexane and acetone at a mass ratio of 2:3 twice for 10 h;
washing was performed using ethanol at a concentration of 99.5% for 0.8 h; and
washing was performed using ethanol at a concentration of 60% at a temperature of 20°C for 12 h.

The degreased tissue was decellularized:
washing was performed using a phosphate-buffered saline at pH 8 twice for 0.8 h each time;
washing was performed using a solution containing 10 mmol/L tris(hydroxymethyl)aminomethane and 8 mmol/L ethylenediaminetetraacetic acid for 2 h;
washing was performed using a solution containing sodium lauryl sulfate at a mass percentage of 1% twice for 12 h each time;
washing was performed using a phosphate-buffered saline containing 8 mmol/L sodium deoxycholate twice for 1 h each time; and
washing was performed using a phosphate-buffered saline twice for 1 h each time.

Nucleic acids were removed from the degreased tissue:
washing was performed using a buffer containing 45 mmol/L tris(hydroxymethyl)aminomethane and 8 mmol/L magnesium ion-containing magnesium salt at pH 8 for 1 h; and
washing was performed using a solution, at pH 8, containing 45 mmol/L tris(hydroxymethyl)aminomethane, 8 mmol/L magnesium ion-containing magnesium salt, bovine serum albumin at a mass percentage of 0.1% and 5,000 U/L Omnipotent nuclease at a temperature of 38°C for 25 h.

After the nucleic acids were removed, the tissue was washed and freeze-dried:
washing was performed using a solution containing 45 mmol/L tris(hydroxymethyl)aminomethane at pH 8 twice for 1 h each time;
washing was performed using a phosphate-buffered saline at pH 8 twice for 1 h each time;
washing was performed using double distilled water twice for 1 h each time; and freeze-drying was performed to obtain a collagen scaffold.

A person skilled in the art can understand that, without conflict, the foregoing preferred solutions can be arbitrarily combined.

It should be understood that the foregoing embodiments are only exemplary rather than restrictive, and a person skilled in the art can make various obvious or equivalent modifications or replacements to the foregoing details without departing from the basic principles of the present invention, which all fall within the scope of the claims of the present invention.

## Claims

1. A method for preparing a collagen scaffold, comprising the following steps:
S100, cleaning a raw material tissue;
S200, performing freeze-thaw cycle treatment on the cleaned raw material tissue to obtain a first intermediate;
S300, degreasing the first intermediate to obtain a second intermediate;
S400, decellularizing the second intermediate to obtain a third intermediate;
S500, removing nucleic acids from the third intermediate to obtain a fourth intermediate; and
S600, washing and drying the fourth intermediate to obtain the collagen scaffold,
wherein in the step S500, the nucleic acids are removed using an omnipotent nuclease solution with a content of 100 U/L to 10,000 U/L.

2. The method for preparing a collagen scaffold according to claim 1, wherein in the step S200, the cleaned raw material tissue is added to a buffer containing tris(hydroxymethyl)aminomethane and phenylmethylsulfonyl fluoride at pH 7.8 to 8.2 to perform the freeze-thaw cycle treatment.

3. The method for preparing a collagen scaffold according to claim 1, wherein the step S300 comprises the following steps:
S310, washing the first intermediate using ethanol in a low-to-high concentration gradient;
S320, degreasing the tissue washed in the step S310 using acetone and hexane; and
S330, washing the tissue treated in the step S320 using ethanol in a high-to-low concentration gradient to obtain the second intermediate.

4. The method for preparing a collagen scaffold according to claim 3, wherein the step S310 comprises the following steps:
S311, washing the first intermediate using ethanol at a concentration of 50% to 70% to obtain an intermediate I; and
S312, washing the intermediate I using ethanol at a concentration of 99% to 100% to obtain an intermediate II.

5. The method for preparing a collagen scaffold according to claim 4, wherein the step S320 comprises the following steps:
S321, extracting polar fats from the intermediate II using acetone to obtain an intermediate III; and
S322, extracting non-polar fats from the intermediate III using a mixture of hexane and acetone to obtain an intermediate IV.

6. The method for preparing a collagen scaffold according to claim 5, wherein the step S330 comprises the following steps:
S331, washing the intermediate IV using ethanol at a concentration of 99% to 100% to obtain an intermediate V; and
S332, washing the intermediate V using ethanol at a concentration of 50% to 70% to obtain the second intermediate.

7. The method for preparing a collagen scaffold according to claim 1, wherein the step S400 comprises the following steps:
S410, washing the second intermediate using a phosphate-buffered saline at pH 7 to 8.2 to obtain an intermediate A;
S420, washing the intermediate A using a solution containing tris(hydroxymethyl)aminomethane and ethylenediaminetetraacetic acid to obtain an intermediate B;
S430, washing the intermediate B using a solution containing sodium lauryl sulfate to obtain an intermediate C;
S440, washing the intermediate C using a phosphate-buffered saline containing sodium deoxycholate to obtain an intermediate D; and
S450, washing the intermediate D using a phosphate-buffered saline to obtain the third intermediate.

8. The method for preparing a collagen scaffold according to claim 1, wherein the step S500 comprises the following steps:
S510, washing the third intermediate using a buffer containing tris(hydroxymethyl)aminomethane and a magnesium salt at pH 7.8 to 8.2 to obtain an intermediate a; and
S520, washing the intermediate a using a solution containing tris(hydroxymethyl)aminomethane, a magnesium salt, bovine serum albumin and omnipotent nuclease at pH 7.8 to 8.2 at a temperature of 30°C to 42°C to obtain the fourth intermediate.

9. The method for preparing a collagen scaffold according to claim 1, wherein the step S600 comprises the following steps:
S610, washing the fourth intermediate using a solution containing tris(hydroxymethyl)aminomethane at pH 7.8 to 8.2 to obtain an intermediate b;
S620, washing the intermediate b using a phosphate-buffered saline at pH 7 to 8.2 to obtain an intermediate c;
S630, washing the intermediate c using double distilled water to obtain an intermediate d; and
S640, freeze-drying the intermediate d to obtain the collagen scaffold.

10. A collagen scaffold, prepared by the preparation method according to any one of claims 1 to 9.
